Europäisches Patentamt

European Patent Office

Office européen des brevets

(11) Publication number: **0 364 580**

**A1**

## (12) EUROPEAN PATENT APPLICATION
### published in accordance with Art. 158(3) EPC

(21) Application number: **88904660.3**

(22) Date of filing: **31.05.88**

(86) International application number:
**PCT/JP88/00534**

(87) International publication number:
**WO 88/09612 (15.12.88 88/27)**

(51) Int. Cl.5: **A01H 1/00 , A01H 5/00 , C12N 5/00**

(30) Priority: **01.06.87 JP 134970/87**

(43) Date of publication of application:
**25.04.90 Bulletin 90/17**

(84) Designated Contracting States:
**IT**

(71) Applicant: **HOKKO CHEMICAL INDUSTRY CO. LTD.**
**4-20, Nihonbashi Hongokucho 4-chome**
**Chuo-ku Tokyo 103(JP)**

(72) Inventor: **TERAKAWA, Teruhiko**
**Casa Miya 205 723-1, Aiko**
**Atsugi-shi Kanagawa-ken 243(JP)**
Inventor: **SATO, Kiyoshi**
**200-96, Higashi Tahara Hatano-shi**
**Kanagawa-ken 257(JP)**

(74) Representative: **Goddar, Heinz J., Dr. et al**
**FORRESTER & BOEHMERT**
**Widenmayerstrasse 4/I**
**D-8000 München 22(DE)**

(54) METHOD OF PRODUCING HERBICIDE-RESISTANT RICE PLANT, SAID RICE PLANT AND SEED OF SAID RICE PLANT.

(57) This invention relates to a method of producing a herbicide-resistant rice plant which comprises cultivating a callus comprising a cultured cell de dedifferentiated from a tissue of a rice plant in a plant tissue culture medium containing a herbicide added thereto, selecting and taking out a mutant of the callus having herbicide resistance proliferated in the medium containing the herbicide, transferring the mutant to a redifferentiation medium to redifferentiate into a young rice plant, cultivating the redifferentiated young rice plant, and selecting and collecting a plurality of herbicide-resistant seeds from the fructified rice plant. This invention also provides a herbicide-resistant rice plant or a seed of the same.

SPECIFICATION

METHOD OF BREEDING RICE PLANTS OF NEW VARIETY
HAVING RESISTANCE TO HERBICIDES, AND THE RICE
PLANTS OF SUCH VARIETY OR SEEDS THEREOF

### TECHNICAL FIELD

This invention relates to a method of breeding or producing such rice plants of a variety having a resistance to a herbicide, which comprises culturing calluses that are composed of the dedifferentiated cultured cells of a tissue of a rice plant, in a plant tissue culture medium containing a herbicide added, selecting and recovering some mutant cells present in the calluses whose cells have been propagated in the medium containing the herbicide, transplanting the mutant cells to a redifferentiation medium, culturing the mutant cells to produce the redifferentiated rice plant bodies, and selecting and harvesting such rice seeds having acquired a resistance to the herbicide from the redifferentiated rice plant bodies.

This invention also relates to the rice plants of a variety having a resistance to a herbicide as bred by the above method, and seeds of said rice plants.

### BACKGROUND ART

Some techniques are hitherto known to breed or produce crop plants having a resistance to herbicide and these techniques comprise using and culturing such calluses which are composed of the dedifferentiated culture cells of a plant, selecting mutant cells having a resistance to herbicide from the cultured calluses, and incubating the mutant cells to produce a crop plant having a resistance to herbicide. For example, example of successful result of providing herbicide-resistant tabacco plants by application of the above techniques to tabacco plants which belong to Solanaceae of dicotyledon plants are described in some publications (a), (b) and (c) mentioned below.

(a) Tabacco plants showing a resistance to a herbicide "Picloram"; see R.S. Chaleff et al's article "Proceeding of the National Academy of Sciences of the United States of America", 75, 5104-5107 (1978) and U.S. Patent No. 4,443,971 specification.

(b) Tabacco plants showing a resistance to a herbicide "Chlorsulfuron"; see R.S. Chaleff et al's article "Science" 223, 1148-1152 (1984).

(c) Tabacco plants showing a resistance to a herbicide "Amitrole"; see Rivks Barg's article "Zeitschrift für Pflanzenphysiologie", 83, 437-447 (1977).

One of the reasons why the application of the above techniques to tabacco plants belonging to Solanaceae has been successful is lying in that successive cultivations of the calluses which are composed of the culture cells of tabacco plants are easy, and it also depends on that the redifferentiation of the calluses of tabacco plant cells into the tabacco plant bodies is easy.

On the other hand, previous investagations on the tissue-culture techniques wherein the calluses of a tissue as derived from a monocotyledon plant of Gramineae such as rice, maize, barley, wheat, etc. are formed and these calluses are redifferentiate to regenerate and produce the normal plant body have not yet led to successful results, unlike to the case of the tabacco plant which is of dicotyledons belonging to the Solanaceae. Especially, little or no example of getting the success are previously reported in the technology of redifferentiating to plant bodies such mutant cell masses which are obtained by applying a herbicidal selection pressure to calluses as formed by dedifferentiating the cells of a monocotyledon plant. In particular, examples of successful results are limited when there is applied such a technique wherein calluses derived from the cultured cells of a monocotyledon plant are cultured in a selection medium containing a herbicide added; and such cell masses which can have survived as a minority even after the culturing stage and exhibit a resistance to said herbicide are taken and used for their redifferentiation into normal plant bodies; and with respect to rice plants, however, there is at all no report of the success in producing the harbicide-resistant rice plant bodies by redifferentiating the calluses that are the dedifferentiated cultured cells of a rice plant. For instance, in Japanese patent application first publication "Kokai" No. 210929/85 specification (or the corresponding European patent application publication No. 154,204 A2 specification), there is described a method for breeding or producing a certain herbicide-resistant plants which comprises using such calluses that are the dedifferentiated culture cells derived from certain plants of monocotyledons of Gramineae and redifferentiating such calluses into plant bodies; and a working Example with maize is given there as an illustrative example. In the Japanese or European patent application publication gazette supra, it is described as if the method disclosed there is applicable also to rice plants. However, neither any enabling disclosure nor working example is found therein about an application of said method to rice plants. Further, there is given at all neither description nor suggestion about a virtually effective method of breeding rice plants of a variety showing a resistance to herbicides.

Furthermore, it has been found to be difficult to breed the herbicide-resistant rice plants when there is employed the under-mentioned other conventional methods of producing an improved variety of rice plants which comprise treating rice plants or seeds thereof with mutagenic agent or crossing rice plants or seeds thereof, without being relied on the method described hereinbefore wherein the calluses of dedifferentiated cultured cells of a plant are tissue-cultivated and the calluses are then redifferentiated into normal plant bodies. For example, with the method of breeding rice plants in which mutations are obtained by irradiating seeds or bodies of rice plants with X rays, etc., the control of direction of occurrence of the mutation involved and the reproducibility of the mutation occurred are not reliable, but there is a possibility that undesirable great changes might be accompanied in the genetically favorable characteristics which are inherent to the existing rice plant varieties. Thus, it was impossible to impart only the herbicide-resistant character preferentially to rice plants. Also with the hybrid-breeding methods in which fertilization is made between different strains of the rice plants, the similar difficulty was observed in producing a herbicide-resistant rice plant.

As has been described hereinbefore, there are known the methods for breeding tabacco or maize plants having a resistance to herbicides wherein calluses composed of dedifferentiated cultured cells of a tabacco or maize plant are subjected to the application of a herbicidal selection pressure and the resulting cell masses of the mutation strain are redifferentiated into normal plant bodies. Further, with respect to rice plants, methods of producing herbicide-resistant rice plants have been investigated and tested in various ways.

However, there is no reported fact that rice plants of a variety having a resistance to a herbicide were obtained with utilizing the callus cells of a tissue of rice plant, and besides, any practicable method which can make breeding of such rice plants of a variety having a resistance to herbicide is yet unknown. A possible major cause for which the breeding of such rice plants of a variety having a resistance to herbicide with using callus cells of rice plant has hitherto failed is probably that the callus cells of rice plants can have a very much lower redifferentiation capability, as compared to the callus cells of other crop plants (such as tabacco and maize), so that no body can find such methods and conditions which are necessary and suitable for performing the effective redifferentiation of the callus cells of rice plants.

On the other hand, when the soil is treated by application of herbicide in the cultivation fields of rice plant for the herbicidal purpose or when the foliages of weeds are applied with herbicide, it often can result in phytotoxic damages in rice plants by the herbicidal compound used.

Accordingly, investigations have been made to use herbicides under such conditions that can minimize the phytotoxic damages in rice plants by the herbicidal agent used. However, many of the available herbicides, because of their potent herbicidal activity, can cause the phytotoxic damages in rice plants even when they are applied by such procedure which is unlikely to involve the phytotoxic damages to rice plants. Further, even such herbicides that normally causes no phytotoxic damages to rice plants through their ordinary application can sometime cause unexpectedly the phytotoxic damages to rice plants, depending upon uneven application of the herbicide, temperature, conditions of soil, etc.

Furthermore, when use is made of such herbicides which normally can cause no phytotoxic damages to the transplanted rice plants, most of such herbicides are likely to cause the damages to the rice plants to kill in some occasion, if the paddy field soil is pre-treated with such herbicides upon or immediately after sowing the rice seeds in the direct planting method for cultivation of rice plants.

Under such situations, hithertobefore, it has been demanded to breed or produce such rice plants of a novel variety having acquired a high resistance to a herbicide in both the transplanting methods for the cultivation and the direct planting method for cultivation of rice plants. An object of this invention is to provide a method of breeding rice plants of a new variety having acquired a resistance to a herbicide, in order to meet the above demands.

## DISCLOSURE OF THE INVENTION

As the results of various investigations, we, the inventors, have now found that rice plants having a resistance to herbicide can be bred and produced by a new method which comprises applying the herbicidal selection pressure to such calluses composed of dedifferentiated culture cells of rice plants, that is to say, culturing such calluses in a suitable culture medium containing such a herbicidal compound added which can causes phytotoxic damages to rice plants, and screening only such a few members of the mutant cells having a resistance to the herbicidal compound which have survived as the minority in said herbicidal culture medium, culturing the mutant cells in a suitable redifferentiation culture medium under the selected conditions, to redifferentiate these cells into normal rice plants, thereby regenerating rice plant bodies, and selecting and harvesting such seeds of rice plants having a resistance to the herbicide from the redifferentiated rice plants.

Thus, according to the present invention, there is provided a method of breeding rice plants of a new variety having a resistance to a herbicide, which comprises the following plural steps.

That is, according to the present invention, there is provided a method of breeding a rice plant of such a variety having a resistance to a herbicide, characterized in that said method comprises (1) a step of forming a plurality of calluses composed of rice plant culture cells which have been dedifferentiated, (2) a step of dividing each of these calluses immediately after the formation of the calluses without performing any successive sub-culturing of the calluses, thereby affording a large number of small masses or small pieces of the callus, transferring a large number of the small masses or small pieces of the callus on such a selection medium which is composed of a plant tissue culture medium (preferably, a plant tissue culture medium as known to be the basic medium N6 or basic medium MS) containing further a herbicide and a plant growth hormone (preferably 2,4-D) added thereto, culturing the small masses or small pieces of the callus and then selecting from the so transferred, inoculated and cultured small masses or small pieces of callus one or more of such callus-like cell mass or masses which has or have received a mutation to acquire a resistance to the herbicide and which has or have been formed by cell-division and propagation of the callus cells, (3) a step of transplanting the so selected callus-like cell mass or masses onto such a redifferentiation medium which is composed of a plant tissue culture basic medium (preferably, the basic medium N6) containing saccharose added thereto at a concentration of 30 to 60 g/ℓ and containing one or more cytokinins added thereto at a concentration of higher than 3 mg/ℓ and up to 10 mg/ℓ, followed by culturing and allowing the callus-like cell mass or masses to grow and redifferentiate in the redifferentiation medium, to produce young rice plant body or bodies, (4) a step of transplanting the thus redifferentiated young rice plant body or bodies from the redifferentiation medium to a medium of paddy field soil, further cultivating the transplanted rice plant body or bodies in the paddy field soil medium by a conventional process for the rice plant cultivation, collecting a plurality of rice seeds of the next generation, namely the second generation from the cultivated rice plant body or bodies which has or have given heading and fertilized rice seeds, followed by sowing a plurality of these seeds of the second generation in such a culture medium for assaying the rice plant's resistance to the herbicide, which has been prepared by adding the same kind of the herbicide as used in the formulation of the aforesaid selection medium into a culture medium or a cultivating soil, incubating the sown seeds and then discarding such seeds which do not germinate and strike root normally in the assaying medium containing the herbicide therein, but selecting such herbicide-resistant seeds of such variant which can have germinated and striked root normally in said assaying medium, and (5) a step of further cultivating the thus selected germinated seeds by the conventional process for the rice plant cultivation, and recovering rice seeds of the third generation from the rice plant bodies which have grown from the selected and cultivated seeds of the second generation and have given heading and fertilized the seeds, to provide the rice seeds of the third generation which are capable of producing rice plants or rice seeds having a resistance to the herbicide.

According to a further aspect of this invention, there is provided a rice plant which is of a variety having a resistance to a herbicide and which is obtained from the method of this invention as described above, and according to another aspect of this invention, there is provided a rice plant seed which is resistant to a herbicide and which is produced by the method of this invention as described above.

According to the method of the present invention, a novel variety of rice plants having a resistance to an application of herbicide can be bred or produced. In the present method, a variety of the original rice plant from which the calluses used as the starting material are to be formed is not particularly limited, but any variety of rice plant which is sensitive to herbicides and readily suffers from the phytotoxic damages by herbicides is usable. The rice plants of a new variety as obtained according to this invention not only have immediately taken over the characteristics of the original variety of rice plant employed, but also have acquired the desired properties of showing the resistance to herbicides.

In order to breed rice plant of such a herbicide-resistant variety, the respective steps (1) through (5) as set out in the above are conducted in accordance with the method of this invention and then have the following functions of them. Thus, briefly speaking:-

At the first step (1), the calluses are formed by dedifferentiating cells of a tissue of rice plant.

At the second step (2), such surviving and growing mutant calluses having a resistance to a herbicide are selected and separated through the culturing of small pieces or small masses of the calluses of the first step in a selection medium.

At the third step (3), the calluses having the resistance to herbicides are redifferentiated to regenerate normal rice plant bodies.

At the fourth step (4), the regenerated rice plant bodies are cultivated to fertilize the seeds, followed by harvesting a number of the seeds of the next or second generation ($R_2$-seeds). From the $R_2$-seeds so harvested are selected such seeds which exhibit the resistance to herbicide and are capable of germinating and striking the root, whereby such $R_2$-seeds having acquired the resistance to herbicide are obtained.

At the fifth step (5), the seeds of the second generation ($R_2$-seeds) which have been selected in the above fourth step and are capable of germinating and striking the root even in the presence of the herbicide and thus exhibit the resistance to herbicide are further cultivated. From the panicles of the rice plants which have thus been cultivated to fertilize the seeds are harvested such seeds of the third generation ($R_3$-seeds) which are capable of providing the herbicide-resistant rice plants as desired according to this invention.

Different culture media are used in the respective steps of the method of the present invention. Thus, the culture medium which is prepared by incorporating a carbon source (for example, saccharose) and one or more of plant growth hormones (cytokinins or auxins) in the MS medium or N6 medium, etc., known as the plant tissue culture basic media is used as the culture medium for forming the calluses in the first step of the present method. At the second step, the culture medium used here is such a selection medium in which the herbicide-resistant calluses are preferentially cultured and grown, and which is prepared by adding herbicidal active compound(s) to a culture medium of such composition that is usable in the first step. At the third step, the culture medium used here is the medium for redifferentiating the herbicide-resistant calluses which have survived as the minority in the selection medium after the second step, whereby rice plant bodies are regenerated. This redifferentiation medium is prepared by adding saccharose as the carbon source and especially one or more cytokinins as the plant growth hormones to the plant tissue culture basic medium.

At the fourth step, the medium used here is the culture medium for assaying the rice plant's resistance to the herbicide (the medium containing herbicide similarly to the selection medium used in the second step), whereby the $R_2$-seeds which exhibit the resistance to herbicides are screened and recovered.

At the fifth step, the medium used here is such a culture medium or a cultivating soil which contains or does not contain any herbicide and in which a conventional process for cultivation of rice plants is carried out in order to cultivate such seeds having the herbicide-resistant property that are capable of germinating and striking the root even in the presence of the herbicide and have germinated and striked the root.

BEST MODE FOR WORKING THE INVENTION

It is preferred that the first to fifth steps of the method of breeding rice plants of a variety having a resistance to herbicide according to the present invention is performed by the following procedures.

(A)  First step (Formation of calluses composed of cultured cells of rice plant)

According to the method of the present invention, calluses are formed from cultured cells of a tissue of a rice plant. The cultured cells of a rice plant used herein can be readily obtained by the known method shown below.

That is, cells of a tissue of a rice plant such as full-mature seeds, immature seeds, anthers, pollens or roots of a rice plant are immersed in an alcohol etc., for example, aqueous 70% to 80% ethyl alcohol for 10 to 60 seconds (for the surface sterilization) and then in an aqueous sodium hypochlorite solution (having a chlorine concentration of about 1%) for an hour to make a sterilization treatment. Thereafter, the sterilized cells of the rice plant tissue described above are inoculated on such a callus-inducing medium. This medium may be such a cullus-inducing medium which is prepared by incorporating 0.5 to 5 ppm, preferably 1 to 2 ppm of an auxin such as 2,4-dichlorophenoxyacetic acid (2,4-D), indoleacetic acid (IAA), naphthaleneacetic acid (NAA), indolebutyric acid (IBA), etc., preferably 2,4-D, as a plant growth hormone and 20 to 40 g/ℓ, preferably 30 g/ℓ of saccharose as the carbon source into MS-agar medium (simply referred to as MS medium) or N6-agar medium (likewise referred to as N6 medium), etc., which are known as the plant tissue culture basic media. Detailed explanation is given hereinafter. When the tissue cells after the inoculation are cultured at a culture temperature of 20 to 30°C, preferably of 28°C, for 20 to 50 days, preferably for 30 to 40 days, the calluses can be formed.

(B)   Second step (Selection of the herbicide-resistant callus)

The calluses thus obtained are divided into small masses or small pieces each having a weight of 10 to 20 mg and a large number of the divided small masses or the small pieces of the calluses are transferred in the selection medium containing herbicide(s).  Some of the small masses or small pieces of the callus used herein can involk mutation by being incubated in the presence of the herbicidal active component(s), thereby acquiring the resistance to the herbicide(s).  It is difficult to say that the rate of occurrence of the mutation is always constant, but it is general that amongst the whole members of the small masses or small pieces of the callus as transferred, some members of the callus pieces, of which the number corresponds to a rate of approximately 0.1 to 0.5% against the total number of the transferred callus pieces, can mutate and consequently can grow during the incubation even in the presence of the herbicide with accompanying the division and propagation of the cells.  Therefore, the number of small masses of calluses required to be transferred for one run is in a range of 5,000 to 20,000, preferably 10,000 to 15,000.  As these small masses of calluses may be used such those as derived from any of full-mature seeds, immature seeds, anthers, pollens or roots of a rice plant, but in general, they may preferably be derived from full mature seeds or anthers. Further, the calluses as derived from the different origins can be used in optional combination. A point to require a special attention in obtaining such small masses of the callus is that a reduction in the redifferentiating ability of the callus as derived from rice plant must be avoided. This is because the redifferentiating ability of the callus of rice plant can usually be reduced with repeated successive sub-cultivation of the callus of rice plant. For this reason, when the calluses have been formed in the first step, it is necessary to directly transplant them to the herbicide-containing selection medium for screening the herbicide-resistant strains of calluses, without sub-culturing them successively.

As the selection medium used in the second step, it is preferred to use such a medium which is prepared by adding to the MS medium or N6 medium such herbicidal active component(s) that can lead to strong phytotoxic damages against rice plants (for example, Bensulfuron methyl in common name, that is, methyl 2-{[(4,6-dimethoxypyrimidin-2-yl)aminocarbonyl]aminosulfonyl-methyl}-benzoate in chemical name, or, for example, glyphosate in common name, that is, N-(phosphonomethyl)-glycine isopropyl amine salt in chemical name) at a concentration of 5 to 500 ppm, preferably about 50 ppm, (though this concentration varies depending upon kind of the herbicidal components), and adding 2,4-D etc., as a plant growth hormone in a range of 0.5 to 5 ppm (preferably 1 to 2 ppm) similarly to the first step and also adding saccharose as a carbon source in an amount of 20 to 40 g/ℓ, preferably 30 g/ℓ.

In order to incubate the small masses or small pieces of the callus and select the herbicide-resistant strains of the callus, the aforesaid selection medium is firstly charged in a Petri dish (preferably, a dish having a diameter of 9 cm) and the small masses of the calluses are inoculated to said medium at a rate of about 50 masses per dish. When the calluses are incubated, most of the calluses are killed owing to the presence of the herbicide because they are sensitive to the herbicide, but a very few members of the calluses can mutate and show the resistance to herbicide. Such surviving calluses can grow with accompanying the cell-division and propagation, thereby forming callus-like cell masses. It is preferred or rather necessary to ensure that a time period elapsed between the transfer of the small masses of callus to the selection medium and the formation of the callus-like cell masses having the herbicide-resistant property should generally be 30 to 90 days, preferably 40 to 50 days. In case that the time period is longer, there is a danger that the callus-like cell masses might not be redifferentiated to the rice plant bodies in the third step. It is thus important to control the aforesaid time period to be 30 to 90 days.

(C)   Third step (Redifferentiation from callus-like cell masses to rice plant)

In order to redifferentiate the surviving cell masses of callus having survived and grown in the selection medium in the second step and showing the resistance to herbicide and thereby to regenerate the rice plant bodies, a medium for effecting the redifferentiation into rice plant bodies as described below is charged in a Petri dish having a diameter of 9 cm, and the surviving cell masses of callus are transplanted to and cultured on the redifferentiation medium. As the medium for effecting the redifferentiation into rice plant bodies, it is preferred to use such culture medium which is prepared by incorporating one or more cytokinins (for example, kinetin, benzyladenine and zeatin) as the plant growth hormones in an amount ranging from 3 to 10 mg/ℓ, preferably 3 to 6 mg/ℓ, and 20 to 90 g/ℓ, preferably 30 to 60 g/ℓ of saccharose as the carbon source into the MS medium or N6 medium.

In order to redifferentiate the callus-like cell masses into rice plant bodies in this redifferentiation medium, light irradiation is required. A light of generally 200 to 2000 lux, preferably 500 to 1000 lux may be irradiated. When the callus-like cell masses are allowed to grow in the above manner, the callus-like cell masses bring about differentiation of green sprouts in about 10 days. In about 20 days subsequent thereto, the cell masses are redifferentiated into rice plant bodies of 10 to 15 cm height which further grow.

If desired, the rice plant bodies as regenerated may be transplanted to a test tube (preferably, a test tube having a diameter of 3 cm and a height of 20 cm) containing a further fresh aliquot of the redifferentiation medium or an agar medium. When cultured for 1 to 2 weeks in this way, the rice plant bodies further grow to get a grass height of about 20 cm and a root of about 5 to 10 cm, thereby providing rice plant seedlings which are well adaptable for their transplantation and affirmative growth in pots containing the paddy field soil, without involving their blighting after the transplantation.

(D) Fourth step (Recovery of seeds of the second or next generation ($R_2$-seeds) having acquired the resistance to herbicide from the regenerated rice plant body)

In order to obtain the $R_2$-seeds, that is, the seeds of the second or next generation from the redifferentiated rice plant bodies as produced in the above step, the following method is preferred.

Firstly, the rice plant bodies as redifferentiated in the third step are transplanted to a Wagner's pot having a size of 1/5000 ares and containing a paddy field soil, followed by culturing the rice plants in a conventional manner until the rice plants come into panicles and fertilize seeds. Then, a plurality of the seeds ($R_2$-seeds) are collected. Next, it is examined by the following method whether or not these seeds have acquired the resistance to herbicide. That is, the $R_2$-seeds described above are aseptically sowed at a rate of one seed per a test tube on the medium for assaying the rice plant's resistance to herbicide, which has been charged in the test tube (diameter of 2 cm and a length of 18 cm), and which medium has been prepared by adding the same herbicidal component as used in the selection medium for the second step to the MS medium or N6 medium or a cultivating soil at a herbicide concentration in a range of 5 to 500 ppm, preferably of 50 ppm (namely, the same herbicide concentration as employed in the second step). The sown seeds are then incubated to grow for 10 to 20 days under light irradiation conditions of about 500 to 1000 lux. Thus, if the tested

$R_2$-seeds include such $R_2$-seeds which have acquired resistance to the herbicide, such herbicide-resistant $R_2$-seeds can normaly germinate and strike root even in the assaying medium containing the herbicidal active component, and therefore it is ready to confirm whether or not some $R_2$-seed have acquired the resistance to herbicide.

Among the seeds so tested, the seeds that have not germinated or striked root normally are discarded, and the $R_2$-seeds that have germinated and striked root normally are selected and provided for the next step.

(E)　Fifth step [Recovery of the seeds of the third generation ($R_3$-seeds) from the seeds of the second or next generation ($R_2$-seeds)]

From the culture medium for assaying the rice plant's resistance to herbicide as used in the preceding step are selectively taken out such seeds of the second generation ($R_2$-seeds) which have germinated and striked the root normally and thus exhibit the resistance to herbicide. These selected seeds are then grown to rice plant seedlings (preferably, to such seedlings having a grass height of about 20 cm at 2-3 leaf stage). These young seedlings are subsequently transplanted into Wagner's pots of 1/5000 ares in size containing a paddy field soil, and they are cultivated by a conventional process of cultivating rice plants until the rice plants produce panicles and fertilize seeds. From the rice plant bodies having fertilized the seeds are harvested the seeds of the third generation ($R_3$-seeds).

The novel variety of rice plants which show the resistance to herbicides can be breded through the respective steps of the present method as described above.

Preparation of various culture media which are usuable in the present invention will now be described.

As the various plant tissue culture basic media which are usable in the present invention, namely, each of the culture media for forming the callus, for selection of the herbicide-resistant callus, for the redifferentiation into rice plant bodies and for selection of the herbicide-resistant $R_2$-seeds, there can be effectively used such those media which may be prepared by adding plant growth hormones (auxins and cytokinins) and carbon sources (preferably, saccharose) to the basic media for plant tissue culture as described below. The plant tissue culture basic media which can be used are such those containing inorganic components, vitamins and amino acids. Examples of the inorganic components include ammonium nitrate, ammonium sulfate, potassium nitrate, potassium chloride, magnesium sulfate, magnesium chloride, sodium nitrate, potassium hydrogenphosphate, calcium chloride, sodium dihydrogenphosphate, ferrous sulfate, ferric sulfate, manganese sulfate, zinc sulfate, cobalt chloride, copper sulfate, sodium molybdenate, potassium iodide, boric acid, etc. Examples of the vitamins include thiamine, pyridoxine, nicotinic acid, nicotinic amide, biotin, calcium panthothenate, ascorbic acid, riboflavin and myoinositol, etc. Examples of the amino acids are glycine, asparagine, glutamine, arginine, etc.

As the plant tissue culture basic media containing such various components, mention may be made of the known media, for example, MS agar medium [described in Murashige and Skoog, "Phisol. Plant", 15, 473-479 (1962)], N6 agar medium [described in Chue et al., "Scientia Scinica", 18, 659-663 (1975)], B5 agar medium [described in Gamborg et al., "Exp. Cell Res.", 50, 151-158 (1968)], R2 agar medium [described in Ohira et al, "Plant Cell Physiol.", 14, 1113-1121 (1973)]. Among them, the compositions of the MS agar medium and N6 agar medium are illustrated below.

In the present invention, Examples of using the MS agar medium and N6 agar medium are given, but the present invention is not limited to the use of these two media. Further, the nature of components and proportions of components added which are shown in the following tables are not necessarily limited to those specifically shown in the following tables.

## Table 1

Constituents and their amounts (mg/ℓ) of the MS medium

| | |
|---|---|
| $NH_4NO_3$ | 1650 |
| $KNO_3$ | 1900 |
| $CaCl_2 \cdot 2H_2O$ | 440 |
| $MgSO_4 \cdot 7H_2O$ | 370 |
| $KH_2PO_4$ | 170 |
| $FeSO_4 \cdot 7H_2O$ | 27.8 |
| $Na_2-EDTA$ | 37.3 |
| $MnSO_4 \cdot 4H_2O$ | 22.3 |
| $ZnSO_4 \cdot 7H_2O$ | 8.6 |
| $CoCl_2 \cdot 6H_2O$ | 0.025 |
| $CuSO_4 \cdot 5H_2O$ | 0.025 |
| $Na_2MoO_4 \cdot 2H_2O$ | 0.25 |
| KI | 0.83 |
| $H_3BO_3$ | 6.2 |
| Myoinositol | 100 |
| Nicotinic acid | 0.5 |
| Pyridoxine hydrochloride | 0.5 |
| Thiamine hydrochloride | 0.1 |
| Glycine | 2.0 |
| Agar | 10000 |
| Distilled water (pH 5.6 - 5.8) | (the balance to make the whole volume to 1 liter) |

## Table 2

Constituents and their amounts (mg/ℓ) of the N6 medium

| | |
|---|---|
| $(NH_4)_2SO_4$ | 463 |
| $KNO_3$ | 2830 |
| $KH_2PO_4$ | 400 |
| $MgSO_4 \cdot 7H_2O$ | 185 |
| $CaCl_2 \cdot 2H_2O$ | 166 |
| $MnSO_4 \cdot 4H_2O$ | 4.4 |
| $ZnSO_4 \cdot 7H_2O$ | 1.5 |
| $H_3BO_3$ | 1.6 |
| KI | 0.8 |
| $FeSO_4 \cdot 7H_2O$ | 27.8 |
| $Na_2$-EDTA | 37.3 |
| Nicotinic acid | 0.5 |
| Pyridoxine hydrochloride | 0.5 |
| Thiamine hydrochloride | 1.0 |
| Glycine | 2.0 |
| Agar | 10000 |
| Destilled water (pH 5.6 − 5.8) | (the balance to make the whole volume to 1 liter) |

Next, preparations of the callus-inducing medium and the selection medium for selection of the callus having the resistance to herbicide, which are used in the present invention, may be conducted by adding one of auxins, for example, 2,4-D, IAA, NAA and IBA, etc., preferrably 2,4-D, or if necessary, two or more of them to the aforesaid MS medium or, N6 medium, etc. Further, preparation of the medium for redifferentiating the calluses into rice plant bodies may similarly be effected by adding one or two or more of cytokinins, for example, kinetin, zeatin and benzyladenine to the aforesaid MS medium or N6 medium, etc.

In the present invention, the herbicidal active component is added to the various culture media described above; herein, such herbicides that bring about strong phytotoxic damges especially against rice plants may advantageously be used. Representative examples of the herbicides are herbicides of sulforylurea type, for example, such compounds described in Japanese Patent Application first publication "Kokai" No. 112379/82 (or European patent application publication No. 51466-A) and "Kokai" No. 78980/85, etc. Representative examples of herbicidal compounds usable in the present invention include methyl 2-{[(4,6-dimethoxypyrimidin-2-yl)aminocarbonyl]aminosulfonylmethyl}benzoate in chemical name (Bensulfuron methyl in common name), and ethyl 5-[(4,6-dimethoxypyrimidin-2-yl)-aminocarbonylaminosulfonyl]-1-methylpyrazole-4-carboxylate (NC 311), etc.

In addition, non-selective herbicides of organic phosphorus type described in U.S. Patent No. 3,799,758, for example, isopropylamine salt of N-(phosphonomethyl)-glycine (common name: glyphosate) can also be used as herbicidal compound.

Next, the present invention will be concretely illustrated with reference to the Examples.

Example 1

(A-1)  Example No. 1 of Induction of Callus (the first step)  (formation of calluses from scutellum of full-mature seed of rice plant)

1000 Grains of full-mature seeds of rice plant (variety: Koshihikari) were immersed in 100 mℓ of 70% ethyl alcohol for 1 minute and then in 500 mℓ of an aqueous solution of sodium hypochlorite (1% chlorine concentration) for 1 hour to sterilize the seeds. Then, the sterilized seeds were inoculated at a rate of 10 seeds per dish on a callus-inducing medium (in a Petri dish of a diameter of 9 cm) which was prepared by adding 300 g of saccharose (to a concentration of 30g/ℓ) and 20 mg of 2,4-D (to a concentration of 2 mg/ℓ, 2 ppm) to 10ℓ of the basic medium N6.  When the seeds were incubated at 28°C for 4 weeks, calluses were formed from the scutellum.

(A-2)  Example No. 2 of  Induction of Callus (the first step) (formation of calluses from anther of rice plant

Panicles were withdrawn from 100 leaf sheathes of rice plant (variety: Norin No. 8) before the heading, and the panicles were immersed in 1 liter of 70% ethyl alcohol for 5 seconds for sterilization. The spikelet was cut out at the one-third level from the tip of the spikelet and anthers (about 600,000 particles) which were present in the spikelet were extracted therefrom and were inoculated into a test tube (diameter of 2 cm x length of 18 cm) containing the same callus-inducing medium as described in the above (A-1) at a rate of 300 to 400 anthers per one test tube. When the anthers were incubated at 28°C for 6 weeks, calluses were formed from pollens present in the anthers.

(A-3)  Example No. 3 of Induction of Callus (the first step) (formation of calluses from scutellum of immature seed of rice plant)

1000 particles of the spikelets of immature seeds (at a stage of 5-10 days after the fertilization) of rice plant (variety: Koshihikari) were immersed in 1 liter of 70% ethyl alcohol for 1 minute for sterilization, and then they were washed with 500 ml of sterilized water. From the spikelets, immature seeds were removed out and inoculated at a rate of 10 seeds per dish on a callus-inducing medium (in a Petri dish of a diameter of 9 cm) which was prepared by adding 300 g of saccharose (to a concentration of 30 g/ℓ) and 10 mg of 2,4-D (to a concentration of 1 mg/ℓ, 1 ppm) to 10ℓ of the basic medium MS. When the immature seeds were then incubated at 28°C for 3 weeks, calluses were formed.

(B)  Example of Selection of the calluses having the herbicide-resistant property (the second step)

The calluses which were obtained in the first step (A-1) were cut and divided into small masses each of about 20 mg weighing to afford 10000 small masses of the calluses. The small masses of the calluses were divided into aliquoats (50 masses per dish) and transferred and inoculated in Petri dishes (diameter, 9 cm) containing a selection medium, namely, such a selection medium which was prepared by adding 20 mg of 2,4-D as a plant growth hormone (to 2 mg/ℓ: 2 ppm), 300 g of saccharose as a carbon source (to 30 g/ℓ) and 500 mg of Bensulfuron methyl as a herbicidal component (to 50 mg/ℓ) to 10 liters of the basic medium N6. When the inoculated masses of the callus were cultured at 28°C for 6 weeks, 40 callus-like cell masses which were grown on said selection medium with making the propagation of the cells were obtained (with development of a mutant strain at a rate of 0.4%). These cell masses were selectively harvested for use in the next step.

(C)  Example of Regeneration of rice plant by redifferentiation of callus-like cell masses (the third step)

The callus-like cell masses as obtained in the preceding step (B) were inoculated and transplanted at a rate of 2 masses per one dish, into petri dishes (of diameter of 9 cm) each containing the redifferentiation medium having the composition shown in Table 3 below (namely, the medium having the composition comprising 1 liter of the basic medium N6 + 3 - 10 mg/ℓ of a cytokinin + 30 - 60 g/ℓ of saccharose). Incubation of the callus-like cell masses was conducted in the redifferentiation medium at 28°C under irradiation with light of 1000 lux. After about 10 days of the incubation, green-colored cells began to be formed from the calluses and then were redifferentiated to regenerate small rice plant bodies of a height of about 5 to 10 cm in about one month of the incubation. The small rice plant bodies were separately transplanted to a large test tube (having diameter of 3 cm x length of 20 cm) containing a fresh redifferentiation medium having the same composition as shown in Table 3, and the transplanted small rice plants were further cultivated in the latter medium, whereby the rice plants grew to a grass height of about 20 cm and a root length of 5 to 10 cm. The results of the regeneration of rice plant bodies by redifferentiation of the callus are shown in Table 3.

## Table 3

### Medium for Redifferentiation of Callus into Rice Plant Bodies and Results of Regeneration to Rice Plant Bodies (Test in Petri Dish)

| Composition of Medium for Redifferentiation of callus into Rice Plant body | | | | | | Rate of regeneration of the redifferentiated rice plant body *1 |
|---|---|---|---|---|---|---|
| Basic-Medium | + Cytokinins (mg/ℓ) | | | + Saccharose (g/ℓ) | | |
| Medium N6 | + Kinetin | 10 | + Saccharose | 60 | | o |
| " | + " | 10 | + " | 30 | | Δ |
| " | + " | 6 | + " | 60 | | o |
| " | + " | 6 | + " | 30 | | Δ |
| " | + " | 5 | + " | 60 | | o |
| " | + " | 5 | + " | 30 | | Δ |
| " | + " | 4 | + " | 60 | | Δ |
| " | + " | 4 | + " | 30 | | Δ |
| " | + " | 3 | + " | 60 | | x |
| " | + " | 3 | + " | 30 | | x |

| Medium N6 + | Benzyl-adenine | 10 | + Saccharose | 60 | ○ |
|---|---|---|---|---|---|
| " + | " | 10 | + " | 30 | ○ |
| " + | " | 6 | + " | 60 | ◎ |
| " + | " | 6 | + " | 30 | ○ |
| " + | " | 5 | + " | 60 | ◎ |
| " + | " | 5 | + " | 30 | ○ |
| " + | " | 4 | + " | 60 | ○ |
| " + | " | 4 | + " | 30 | Δ |
| " + | " | 3 | + " | 60 | Δ |
| " + | " | 3 | + " | 30 | Δ |
| Medium N6 + | Zeatin | 10 | + Saccharose | 60 | ○ |
| " + | " | 10 | + " | 30 | ○ |
| " + | " | 6 | + " | 60 | ◎ |
| " + | " | 6 | + " | 30 | ○ |
| " + | " | 5 | + " | 60 | ◎ |
| " + | " | 5 | + " | 30 | ○ |
| " + | " | 4 | + " | 60 | ○ |
| " + | " | 4 | + " | 30 | Δ |
| " + | " | 3 | + " | 60 | Δ |
| " + | " | 3 | + " | 30 | x |

| Medium N6 + None | 0 | + Saccharose | 60 | Δ |
|---|---|---|---|---|
| " + | 0 | + " | 30 | Δ |

*1 : ◎ Well redifferentiated into rice plants of a grass height of about 5-10 cm at a rate of regeneration of the rice plant of 50% or more, as estimated in term of percentages of the number of the redifferentiated callus pieces to regenerate rice plant bodies, against the total number of the callus pieces inoculated.

○ Redifferentiated at a rate of regeneraration of the rice plant of 20 to less than 50%, as estimated as above.

Δ Regenerated at a rate of regeneration of the rice plant of 5% to less than 20%, as estimated as above.

x The rate of regeneration of the rice plant not exceeding 5%.

Further, as the medium N6 was used the one having the composition as described in Table 2.

(D) Example of Collection of the second generation seeds (R₂-seeds) having acquired the resistance to herbicide, from the rice plant bodies (the former-half part of the fourth step)

Thirteen strains of the redifferentiated rice plant bodied (having grass height of about 20 cm, at 2-3 leaf stage) (as obtained from the herbicide-resistant calluses), which were grown in the test tube in step (C) described above, were transplanted to Wagner's pots of 1/5000 ares which was each packed with paddy field soil containing no herbicide. The transplantation was made at a rate of one strain per pot. The rice plant bodies were then cultivated, manipulated and grown by a conventional process of cultivating rice plant. The rice plant bodies gave the heading and fertilized to give seeds. A number of seeds of the next generation ($R_2$-seeds were harvested from the ears of rice plants having fertilized seeds.

(E) <u>Example of Collection of the third generation seeds ($R_3$-seed) from the second generation seeds ($R_2$-seed) (the latter-half part of the fourth step and the fifth step)</u>

(1) Test for confirming that the second generation seeds ($R_2$ generation seeds) show a resistance to herbicides

The $R_2$ generation seeds of the 13 strains which were obtained as described above in the procedure (D) (the number of grains of the seeds tested is as described in Table 4) were immersed in 100 mℓ of 70% ethyl alcohol for 1 minute and then in 100 mℓ of a sodium hypochlorite aqueous solution (the chlorine concentration of 1%) for 1 hour for sterilization. In 1 liter of the medium MS was dissolved 50 mg of Bensulfuron methyl (a herbicidal compound) to prepare a medium for assaying the resistance to herbicide. This assaying medium was poured in 10 mℓ-portions in test tubes (of diameter of 2 cm x length 18 cm).

The sterilized seeds were inoculated into the assaying medium at a rate of one seed per test tube. Germination of the seeds was induced at 28°C under light-irradiating conditions of 1000 lux (with light-irradiation for 12 hours). The test results are shown in Table 4.

Among the seeds as submitted to the germination test, the seeds which germinated and provided the seedlings of rice plant are assumed to have acquired the resistance to the herbicide.

## Table 4

Results of Assay for Resistance to Herbicide
with Seeds of Second Generation (R$_2$-seeds)

| Strain No. of Herbicide-Resistant Plant Body | Total Number of Seeds Tested | Number of Seedlings with Herbicide-Resistance *1 | Number of Seedlings with Herbicide-Sensitivity *2 |
|---|---|---|---|
| 1 | 40 | 12 | 28 |
| 2 | 40 | 14 | 26 |
| 3 | 40 | 16 | 24 |
| 4 | 40 | 18 | 22 |
| 5 | 40 | 14 | 26 |
| 6 | 40 | 8 | 32 |
| 7 | 80 | 50 | 30 |
| 8 | 40 | 14 | 26 |
| 9 | 80 | 61 | 19 |
| 10 | 40 | 18 | 22 |
| 11 | 40 | 18 | 22 |
| 12 | 40 | 20 | 20 |
| 13 | 40 | 10 | 30 |
| Comparative rice plant of variety: Koshihikari | 40 | 0 | 40 |

*1 The rice plant seedlings having the herbicide-resistance, after germination, was observed to grow to such extent that the growth of the seedlings of rice plant took placed to a height of 50 mm or more in the grass part and to a length of 10 mm or more in the root part.

*2 The seeds having the herbicide-sensitivity was observed to be such ones that the germination might occur but the growth of the rice plant took place at:

Grass part: 0 mm

Root part: 0 mm

As the comparative rice plant, an ordinary rice plant of the variety (Koshihikari) which was not treated in accordance with the present invention was used.

(2) Selection of the herbicide-resistant seeds ($R_3$-generation)

The seedlings derived from herbicide-resistant seeds of rice plant that germinated and elongated at the stage (1) described above were cultured in a conventional manner. The seedlings were transplanted to Wagner's pots having a size of 1/5000 ares and then cultivated and manipulated in a manner similar to the illustrative procedure (D) of the fourth step. The third generation seeds ($R_3$ generation) was harvested from the spikes of rice plants having fertilized seeds.

In a manner similar to this method, seedlings of rice plant (comparative Example), which were not treated in accordance with the present invention, were transplanted to paddy field, came into the heading and fertilized to give seeds. When the rice plants in accordance with the comparative Example were compared with the rice plants in accordance with the present invention, there were observed no particular difference between them in respect of their grass height, length of internode, number of panicles, length of panicles, number of tillers and number of rice hulls per spike, total weight (g) of 1000 brown rice grains, time for coming into heading, etc. Accordingly, it is considered that this invention can impart only the resistance to herbicide to conventional variety of rice plant.

(3) Test for confirming that $R_3$-generation seeds show the resistance to herbicide (Run 1: Test for germination in vitro)

Assaying test was made in the same manner as in the method of assay which is shown for Table 4. As shown in the following Table 5, it is found that almost of the $R_3$ generation seeds which were harvested in the above stage (2) completely germinated and grew and have acquired the resistance to herbicide.

## Table 5

### Results of Assay for Resistance to Herbicide with Seeds of Subsequent Generation ($R_3$-seeds)

| Strain No. of Herbicide-Resistant Plant Body ($R_3$-generation) | Total Number of Seeds Tested | Number of Seedlings with Herbicide-Resistance *1 | Number of Seedlings with Herbicide-Sensitivity *2 |
|---|---|---|---|
| 7-1 | 40 | 28 | 12 |
| 7-2 | 40 | 31 | 9 |
| 7-3 | 40 | 40 | 0 |
| 7-4 | 40 | 30 | 10 |
| 7-5 | 40 | 25 | 15 |
| 7-6 | 40 | 31 | 9 |
| 7-7 | 40 | 40 | 0 |
| 7-8 | 40 | 40 | 0 |
| 7-9 | 40 | 28 | 12 |
| 7-10 | 40 | 30 | 10 |
| 9-1 | 40 | 40 | 0 |
| 9-2 | 40 | 30 | 10 |
| 9-3 | 40 | 27 | 13 |
| 9-4 | 40 | 40 | 0 |
| 9-5 | 40 | 32 | 8 |
| 9-6 | 40 | 40 | 0 |
| 9-7 | 40 | 30 | 10 |
| 9-8 | 40 | 28 | 12 |

| | | | |
|---|---|---|---|
| 9-9 | 40 | 31 | 9 |
| 9-10 | 40 | 40 | 0 |
| Comparative Example | 40 | 0 | 40 |

*1   Young seedlings of rice plant having the herbicide-resistance could exhibit the same resultant degrees of germination and elongation as estimated in Table 4.

*2   Seeds having the herbicide-sensitivity gave the same observations as shown in Table 4.

Example 2

Selection of the herbicide-resistant calluses was attempted with using different herbicides.  The selection was carried out in a manner similar to Example 1.  As a result, completely the same results as in Example 1 were obtained as shown in the following:-

(A)   Example of Selection of the herbicide-resistant calluses

The calluses as obtained in Example 1-(A) were cut and divided into small masses of about 20 mg weighing to afford 10,000 small masses of callus.  These small masses were divided into aliquots (50 masses per dish) and inoculated on Petri dishes (diameter, 9 cm) containing such a selection medium, namely, such a selection medium which was prepared by adding 20 mg of 2,4-D as a plant growth hormone (2 mg/ℓ: 2 ppm), 300 g of saccharose as a carbon source (30 g/ℓ) and 1.7 g of Glyphosate as a herbicidal component (170 mg/ℓ) to 10 liters of the basic medium N6. When cultured at 28°C for 6 weeks, 27 callus-like cell masses which were surviving and grown on said medium were harvested and provided for use in the next step.

(B)  Example of Regeneration of rice plant by redifferentiation of callus-like cell masses having the resistance to herbicide, and example of recovery of the second generation seeds ($R_2$-seeds) having acquired the resistance to glyphosate from the rice plant

The callus-like cell masses as obtained in the preceding stage (A) were redifferentiated according to the method described in Example 1-(C). As a result, 10 strains of redifferentiated rice plant bodies were obtained. These 10 strains of the rice plant bodies which were derived from the herbicide-resistant calluses were cultivated in the method described in Example 1-(D). The seeds of the second generation ($R_2$-seeds) could be harvested from the panicles of the rice plants having fertilized seeds.

The results of the test described above are summarized in Table 6.

### Table 6

Results of Selection and Redifferentiation of Glyphosate-Resistant Callus

| Number of Test | Number of Small Masses of Callus Tested | Number of Callus Showing the herbicide-Resistance | Number of Redifferentiated Plant Body obtained from Callus Showing the herbicide-Resistance |
|---|---|---|---|
| 1 | 2405 | 4 | 2 |
| 2 | 2294 | 3 | 1 |
| 3 | 2849 | 13 | 4 |
| 4 | 2516 | 7 | 3 |
| Total | 10064 | 27 | 10 |

Incidentally, the redifferentiation medium employed had a composition comprising the N6 medium + 6 mg/ℓ of benzyladenine + 60 g/ℓ of saccharose.

By performing the method of the present invention, the following effects can be exhibited.

Firstly, there can be bred new varieties of rice plants which are free from symptoms of phytotoxic damages involved due to application of herbicides, for example, growth-inhibition such as inhibition of germination, and blightening of leaf, etc.

Secondly, rice plants of new varieties as by the present invention have received occurrence of a mutation by applying a herbicidal selection pressure to the cultured cells of rice plants so that the occurring mutation enables the rice plants to acquire the resistance to a herbicide. Therefore, the mutation can be involved with a control of direction of the mutation and with reproducibility of the mutation.

Thirdly, the method of the present invention is advantageous in that it can impart the resistance to herbicide alone to rice plants of new variety while genetically good characteristics (for example, taste, yield, etc.) of rice plants of the existing varieties can be kept to remain.

Fourthly, the variety of original rice plants to which the resistance to herbicide is to be imparted by the present invention is not limited specifically and therefore, the present invention has a wide applicability.

Fifthly, the herbicidal active component as used may be such those that cause strong phytotoxic damages to rice plants, for example, compounds of sulfonyl urea type, organic phorphorus compounds and herbicidal active components of a type inhibitory to the division of cells.

Sixthly, such rice plants that have once acquired the resistance to a herbicidal active component likely to cause strong phytotoxic damages can show further a similar resistance also to another herbicidal active components of similar types which are likely to cause similar phytotoxic damages to rice plants. Further, the rice plants having acquired a resistance to a herbicide will be able to exhibit a resistance also against phytotoxic damages which are caused by herbicides of different types, as long as the mechanism of the activity of the active component of the latter herbicides against rice plants is same as that of the former herbicide.

INDUSTRIAL UTILITY OF THE INVENTION

Thus, according to the method of the present invention, it is possible to produce and breed rice plants of a variety having a resistance to herbicides and being useful as described in the above.

CLAIM

1.    A method of breeding a rice plant of such a variety having a resistance to a herbicide, characterized in that said method comprises (1) a step of forming a plurality of calluses composed of rice plant culture cells which have been dedifferentiated, (2) a step of dividing each of these calluses immediately after the formation of the calluses without performing any successive sub-culturing of the calluses, thereby affording a large number of small masses or small pieces of the callus, transferring a large number of the small masses or small pieces of the callus on such a selection medium which is composed of a plant tissue culture medium (preferably, a plant tissue culture medium as known to be the basic medium N6 or basic medium MS) containing further a herbicide and a plant growth hormone (preferably 2,4-D) added thereto, culturing the small masses or small pieces of the callus and then selecting from the so transferred, inoculated and cultured small masses or small pieces of callus one or more of such callus like-cell mass or masses which has or have received a mutation to acquire a resistance to the herbicide and which has or have been formed by cell-division and propagation of the callus cells, (3) a step of transplanting the so selected callus-like cell mass or masses onto such a redifferentiation medium which is composed of a
plant tissue culture basic medium (preferably, the basic
medium N6) containing saccharose added thereto at a
concentration of 30 to 60 g/ℓ and containing one or
more cytokinins added thereto at a concentration of
higher than 3 mg/ℓ and up to 10 mg/ℓ, followed by cultur-
ing and allowing the callus-like cell mass or masses to
grow and redifferentiate in the redifferentiation medium,
to produce young rice plant body or bodies, (4) a step
of transplanting the thus redifferentated young rice
plant body or bodies from the redifferentiation medium
to a medium of paddy field soil, further cultivating the
transplanted rice plant body or bodies  in the paddy
field soil medium by a conventional process for the rice
plant cultivation, collecting a plurality of rice seeds
of the next generation, namely the second generation
from the cultivated rice plant body or bodies which has
or have given heading and fertilized rice seeds, followed
by sowing a plurality of these seeds of the second generation in such a culture medium for assaying the rice
plant's resistance to the herbicide, which has been
prepared by adding the same kind of the herbicide as
used in the formulation of the aforesaid selection medium
into a culture medium or a cultivating soil, incubating
the sown seeds and then discarding such seeds which do not germinated and strike root normally in the assaying medium containing the herbicide therein, but selecting the herbicide-resistant seeds of such variant which can have germinated and striked root normally in said assaying medium, and (5) a step of further cultivating the thus selected germinated seeds by the conventional process for the rice plant cultivation, and recovering rice seeds of the third generation from the rice plant bodies which have grown from the selected and cultivated seeds of the second generation and have given heading and fertilized the seeds, to provide the rice seeds of the third generation which are capable of producing rice plants or rice seeds having a resistance to the herbicide.

2. A method as claimed in claim 1, wherein the callus composed of the dedifferentiated rice plant culture cells is a callus which has been formed by culturing either a scutellum of the rice seed from a rice plant of ordinary variety, or a pollen in an anther of spikelet of panicles as excised from the leaf sheath of the rice plant before it comes into spikes, or a spikelet of immature seed, at 28°C for 4 to 6 weeks in such a plant tissue culture basic medium N6 or basic medium MS containing 1 to 2 ppm of 2,4-D as a plant growth hormone added thereto.

3.    A method as claimed in claim 1, wherein the divided small masses or small pieces of the callus are so transferred to and cultured on the herbicide-containing selection medium that the small callus masses or pieces as placed on said selection medium provide the callus-like cell masses of the variant having a resistance to the herbicide, within a time period of 30 to 90 days, preferably of 40 to 50 days from the beginning of the transfer of the small masses or pieces of the callus.

4.    A method as claimed in claim 1, wherein at least 10,000 small masses or small pieces (each weighing 10 to 20 mg) of the callus are transferred into and cultured in the selection medium, and the cultured callus-like cell mass or masses of a varient having a resistance to the herbicide is or are selectively harvested from the selection medium.

5.    A method as claimed in claim 1, wherein the selection medium is composed of a plant tissue culture basic medium N6 or MS containing 1 to 2 ppm of 2,4-D and 40 to 50 ppm of a herbicide added thereto.

6.    A method as claimed in claim 1, wherein the redifferentiation medium is composed of such a basic medium N6 containing saccharose added at a concentration of 30 to 60 g/ℓ and containing kinetin, benzyladenine or zeatin as cytokinins added at a concentration of higher than 3 mg/ℓ and up to 6 mg/ℓ.

7.    A method as claimed in claim 1, wherein the young rice plants which have been grown and redifferentiated in the redifferentiation medium are optionally transfered into a fresh redifferentiation medium or an agar medium having the same composition as that of the redifferentiation medium initially used, followed by being cultivated in the fresh redifferentiation medium for a period of about 1 to 2 weeks until the rice plants obtain a grass height of about 20 cm and a root length of about 5 to 10 cm, and the rice plants so cultivated are then transplanted to the medium of paddy field soil for their further cultivation.

8.    A method as claimed in claim 1, wherein the concentration of the herbicide present in the medium for assaying the rice plant's resistance to the herbicide on which the seeds of the second generation are to be sown is equal to or higher than the concentation of said herbicide which is present in the selection medium as initially used to transfer the callus of dedifferentiated rice plant culture cells.

9.    A rice plant which is of a variety having a resistance to a herbicide and which is produced by the method of claim 1.

10.    A rice plant seed which is resistant to a herbicide and which is produced by the method of claim 1.

# INTERNATIONAL SEARCH REPORT

International Application No PCT/JP88/00534

| I. CLASSIFICATION OF SUBJECT MATTER (if several classification symbols apply, indicate all) [6] |
|---|

According to International Patent Classification (IPC) or to both National Classification and IPC

Int.Cl[4]  A01H1/00, 5/00, C12N5/00

| II. FIELDS SEARCHED |
|---|

**Minimum Documentation Searched [7]**

| Classification System | Classification Symbols |
|---|---|
| IPC | A01H1/00, 1/04, 5/00, A01G1/00, C12N5/00 |

Documentation Searched other than Minimum Documentation
to the Extent that such Documents are Included in the Fields Searched [8]

## III. DOCUMENTS CONSIDERED TO BE RELEVANT [9]

| Category * | Citation of Document, [11] with indication, where appropriate, of the relevant passages [12] | Relevant to Claim No. [13] |
|---|---|---|
| Y | JP, A, 61-74519 (Saint-Jean Technologies Corp.) 16 April 1986 (16. 04. 86) P3-4, P12 (Family: none) | 1-10 |
| Y | JP, A, 60-210929 (Molecular Genetics, Inc.) 23 October 1985 (23. 10. 85) & EP, A2, 154,204 | 1-10 |
| Y | US, A, 4,443,971 (Cornell Research Foundation Inc.) 24 April 1984 (24. 04. 84) | 1-10 |
| Y | Nihon Dojo Hiryo Kagaku Zasshi Vol.57, No.6, 1986, Okawara Ryoji and two others "Aluminium Taisei Inekarusu no Sentaku to sono Saibunka" P.558-562 | 1-10 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| August 11, 1988 (11. 08. 88.) | August 29, 1988 (29. 08. 88) |

| International Searching Authority | Signature of Authorized Officer |
|---|---|
| Japanese Patent Office | |

Form PCT/ISA/210 (second sheet) (January 1985)